# FASCICULE DE BREVET EUROPEEN

(11) **EP 3 331 517 B1**
(45) Date de publication et mention de la délivrance du brevet: **06.11.2019**
(21) Numéro de dépôt: 16750804.3
(22) Date de dépôt: 08.08.2016
(51) Int. Cl.: A61K 31/17, A61P 27/02

(54) **UTILISATION D'HYDROXYCARBAMIDE POUR PREVENIR LA NON-PERFUSION RETINIENNE**
VERWENDUNG VON HYDROXYCARBAMID ZUR VORBEUGUNG EINER RETINALEN ISCHÄMIE
USE OF HYDROXYCARBAMIDE FOR PREVENTING RETINAL NON-PERFUSION

(30) Priorité: 06.08.2015 EP 15306277
(43) Date de publication de la demande: 13.06.2018
(73) Titulaire: Centre Hospitalier National d'Ophtalmologie Quinze-Vingts, 75012 Paris (FR); Institut National de la Santé et de la Recherche Médicale (INSERM), 75013 Paris (FR); Centre National de la Recherche Scientifique (CNRS), 75016 Paris (FR); Imagine Institut des Maladies Génétiques Necker Enfants Malades, 75015 Paris (FR); Assistance Publique - Hôpitaux de Paris, 75004 Paris (FR); Université Paris Descartes, 75006 Paris (FR)
(72) Inventeur: COLIN, Yves, 92170 Vanves (FR); GIRMENS, Jean-François, 75011 Paris (FR); HERMINE, Olivier, 75014 Paris (FR); HERON, Emmanuel, 92290 Chatenay-malabry (FR); PAQUES, Michel, 75001 Paris (FR)
(74) Mandataire: Regimbeau
(86) Numéro de dépôt international: PCT/EP2016/068908
(87) Numéro de publication internationale: WO 2017/021560

(56) Documents cités:
- WO-A1-00/51620
- US-A1- 2014 171 504
- US-B1- 6 462 071
- SANGHVI C ET AL: "Superior macular sparing in central retinal artery occlusion due to sickle cell anaemia [9]", EYE 200404 GB, vol. 18, no. 4, avril 2004 (2004-04), pages 442-443, XP002752753, ISSN: 0950-222X
- DHRAMI-GAVAZI ELONA ET AL: "Jak2 mutation-positive polycythemia vera presenting as central retinal artery occlusion.", RETINAL CASES & BRIEF REPORTS SPRING 2015, vol. 9, no. 2, avril 2015 (2015-04), pages 127-130, XP009187876, ISSN: 1937-1578
- FARCET J P ET AL: "A HYPER EOSINOPHILIC SYNDROME WITH RETINAL ARTERITIS AND TUBERCULOSIS", ARCHIVES OF INTERNAL MEDICINE, vol. 142, no. 3, 1982, pages 625-627, XP009187862, ISSN: 0003-9926
- JULIE GLANVILLE ET AL: "Efficacy and safety of widely used treatments for macular oedema secondary to retinal vein occlusion: a systematic review", BMC OPHTHALMOLOGY, BIOMED CENTRAL, LONDON, GB, vol. 14, no. 1, 21 janvier 2014 (2014-01-21), page 7, XP021174278, ISSN: 1471-2415, DOI: 10.1186/1471-2415-14-7
- SPAIDE ET AL: "PERIPHERAL AREAS OF NONPERFUSION IN TREATED CENTRAL RETINAL VEIN OCCLUSION AS IMAGED BY WIDE-FIELD FLUORESCEIN ANGIOGRAPHY", RETINA, LIPPINCOTT WILLIAMS AND WILKINS, PHILADELPHIA, PA, US, vol. 31, no. 5, 1 mai 2011 (2011-05-01), pages 829-837, XP008177263, ISSN: 0275-004X, DOI: 10.1097/IAE.0B013E31820C841E

## Description

### Résumé

La présente invention concerne l'utilisation de l'Hydroxycarbamide (HC) pour atténuer et/ou retarder l'extension de la non-perfusion capillaire, cause de baisse visuelle irrémédiable chez les patients souffrant d'occlusion de veine centrale de la rétine (OVCR). Il s'agit ici du premier traitement systémique permettant de réduire les complications ischémiques rétiniennes chez des patients dont l'OVCR a été diagnostiquée récemment et est par conséquent dans une phase de progression rapide. Compte tenu de la faible toxicité de l'HC évaluée à grande échelle chez l'enfant et l'adulte dans le cadre d'autres maladies depuis des décennies, les résultats de la présente étude ouvrent une nouvelle voie thérapeutique dans le traitement de l'OVCR.

### Art antérieur

Les occlusions veineuses rétiniennes (OVR) comprennent les occlusions de veine centrale de la rétine (OVCR) et les occlusions de branches veineuses rétiniennes (OBVR), dont les mécanismes, pronostic et traitements diffèrent (Pierru A et al. EMC - Ophtalmologie, 21-240-E15, 2015). Les OVCR diffèrent des OBVR par leur pronostic plus grave, l'âge moyen de début (10 ans plus jeune), et l'existence de cas familiaux. Au plan physiopathologique, les OBVR surviennent presque constamment au niveau d'un croisement artério-veineux (suggérant l'implication d'un processus local dans sa physiopathologie), alors que, dans le cas des OVCR, la localisation de l'obstruction veineuse est variable et son mécanisme inconnu. La prévalence des OVCR est environ 5 fois plus faible que celle des OBVR.

On distingue également les OVCR des occlusions de l'artère centrale de la rétine (OACR), ces dernières résultant le plus souvent d'une embolie cardiaque ou vasculaire.

Une méta-analyse des données épidémiologiques sur les OVR a été publiée en 2010 (Rogers S. et al. Ophtalmology 2010;117:313-9), avec une estimation de la prévalence moyenne mondiale des OVCR de 0,8 pour 1000, soit 2,5 Millions de personnes affectées. L'OVCR est une affection grave, responsable chez plus de 50% des patients de baisse visuelle sévère pouvant aller jusqu'à la cécité. En effet, une acuité visuelle < 1/10 à 2 ans est observée dans environ 50% des cas (80% si l'acuité visuelle initiale est < 1/10) (Pierru A et al. EMC - Ophtalmologie, 21-240-E15, 2015). De plus, il existe un risque de cécité bilatérale lié à cette affection.

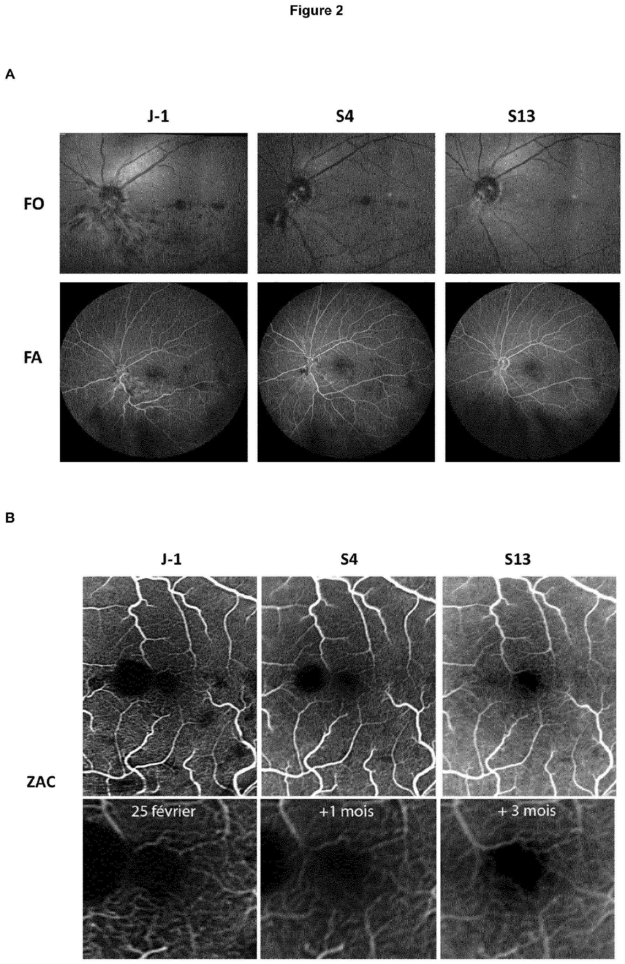

La complication la plus sévère des OVCR est la survenue d'une non-perfusion capillaire rétinienne détectée par la présence d'une absence de perfusion capillaire sur un territoire plus ou moins étendu (voir le point J-1 de la Figure 2), entraînant une ischémie rétinienne responsable non seulement d'une baisse sévère de la vision mais, de plus, d'une prolifération néovasculaire pouvant mener à un glaucome (Pierru A et al. EMC - Ophtalmologie, 21-240-E15, 2015). Le mécanisme de survenue de la non-perfusion capillaire est débattu. Les hypothèses évoquées font appel à un œdème des cellules endothéliales, une apoptose endothéliale, une obstruction leucocytaire, une thrombogenèse locale ou des troubles hémorhéologiques (déformabilité et agrégation érythrocytaires notamment). Si peu de données sont disponibles en ce qui concerne la variation de la surface de non-perfusion, il a été démontré qu'elle est de l'ordre de 25% en moyenne lors de la première angiographie (Singer M et al. Retina 2014; 34:1736-42). La surface de non-perfusion rétinienne chez ces patients s'accroit très rapidement, d'environ 30% dans les premiers 15 jours de la maladie (Singer M et al, Retina 2014;34:1736-42 ; Spaide RF. Retina 2011;31:829-37). De même, les complications de la non-perfusion surviennent en général dans les premiers mois d'évolution de l'OVCR (Sinclair SH et al. Br J Ophthalmol. 1979;63:735-43; Hayreh SS et al. Retina 2012;32:1553-65).

Malgré la place importante des OVCR en pathologie vasculaire de la rétine, leur physiopathologie reste obscure, et très peu de facteurs de risque ont été identifiés.

Par le passé, il a été proposé d'administrer des traitements systémiques antithrombotiques chez des patients souffrant d'une OVCR. Cependant, ces traitements (aspirine, warfarine) se sont révélés inefficaces, voire délétères, chez ces patients (Pierru A et al. EMC - Ophtalmologie, 21-240-E15, 2015; Hayreh SS. et al. Ophtalmology 2011;118:1603-1611). Il apparaît aujourd'hui que, chez les patients souffrant d'OVCR, le phénomène occlusif aigu n'est pas lié à la formation primitive de thrombus, puisque l'angiographie en fluorescence montre un ralentissement plus ou moins marqué du flux veineux sans arrêt véritable (Pierru A et al. EMC - Ophtalmologie, 21-240-E15, 2015), que la veine centrale de la rétine reste perméable au doppler (Williamson TH. Br J Ophthalmol 2007;91:1190-1193), et que la présence de thrombus est inconstante dans les rares cas anatomopathologiques rapportés (Green WR et al. Retina 1981;1:27-55).

Aucun autre traitement systémique n'a démontré son efficacité pour traiter l'OVCR et ses symptômes.

Le plus souvent, lorsqu'une OVCR est diagnostiquée, l'attitude thérapeutique se limite à administrer localement (i.e., dans la cavité vitréenne de l'œil) des anticorps anti-VEGF ou des corticoïdes et/ou à réaliser localement une photocoagulation rétinienne (Glanville J et al. BMC Ophthalmol 2014;14:7). Cette administration locale est pénible pour le patient, qui doit se rendre à l'hôpital à intervalles réguliers, en vue de subir l'injection intra-vitréenne. En outre, cette opération est invasive, puisque l'aiguille doit traverser la conjonctive et la sclère dans la cavité oculaire située en arrière du cristallin. Elle ne peut être réalisée que par un médecin, dans des conditions d'hygiène strictement contrôlées, ce qui restreint son accès aux patients pouvant se déplacer dans les centres équipés pour cette opération. En outre, cet acte chirurgical peut générer d'éventuelles complications : l'infection intraoculaire (qui peut survenir, comme dans n'importe quelle ponction ou acte chirurgical, malgré les précautions rigoureuses d'asepsie), l'hypertonie intra-oculaire (élévation de la pression intra-oculaire), une blessure accidentelle du cristallin, ou encore le décollement de la rétine.

De plus, bien que relativement efficaces pour supprimer la prolifération néovasculaire, aucun de ces traitements locaux ne permet d'empêcher l'extension de la surface de non-perfusion rétinienne, qui est la cause de l'ischémie rétinienne et de ses complications cécitantes (atrophie maculaire, néovascularisation, hémorragies rétiniennes/intravitréennes, glaucome néovasculaire). Il a par exemple récemment été observé que la surface de non-perfusion rétinienne progresse d'environ 30% lors des 3 premiers mois en cas d'OVCR ischémique, même si le patient est traité localement avec des anti-VEGF (Wykoff CC et al. Retina 2015;35:43-7). Les injections d'anti-VEGF n'ont qu'un effet « suspensif », la prolifération néovasculaire pouvant alors survenir après leur arrêt (Brown DM et al. Retina 2014;34:1728-35).

La perte visuelle subie par les patients souffrant d'une OVCR avec ischémie rétinienne est donc considérée aujourd'hui comme irréversible, aucun cas de reperfusion spontanée (même localisée) de l'occlusion capillaire rétinienne n'ayant été documenté à ce jour.

Dans ce contexte, il devenait urgent d'identifier un traitement non-invasif et efficace en vue de traiter les patients souffrant d'OVCR, pour les soulager des symptômes liés à cette pathologie et, plus précisément, pour empêcher l'extension de la surface de non-perfusion rétinienne, responsable de l'atrophie rétinienne observée chez ces patients

### Description de l'invention

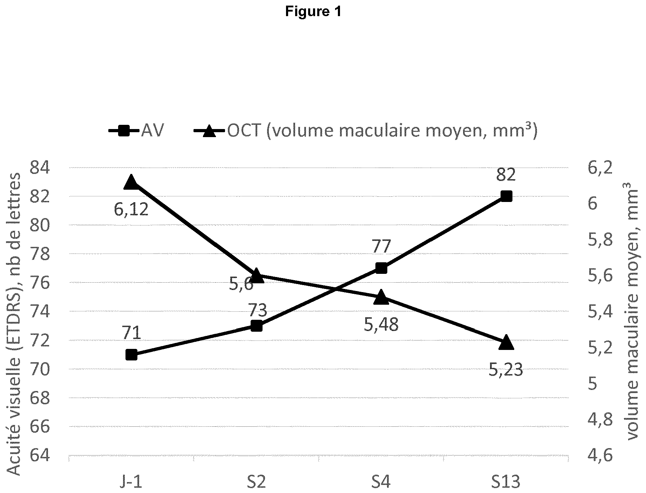

Les présents inventeurs démontrent ici qu'il est possible de réduire l'extension de la surface de non-perfusion rétinienne de façon significative en administrant aux patients souffrant d'OVCR, et notamment ceux souffrant d'une OVCR récemment diagnostiquée (i.e., depuis moins d'un mois) et donc en pleine progression, une dose initiale de 20mg/kg/jour d'hydroxycarbamide (HC) par voie orale (Figures 1 et 2).

L'étude présentée ci-après permet de démontrer, par divers indicateurs cliniques, que la surface de non-perfusion rétinienne est stabilisée après quelques semaines d'un tel traitement (Résultats et Figures 1 et 2).

Ainsi, la présente demande de brevet vise, dans un premier aspect, l'hydroxycarbamide pour son utilisation pour traiter, par voie systémique, les patients souffrant d'OVCR.

Plus précisément, l'hydroxycarbamide peut être utilisée, chez ces patients, pour :
- prévenir ou retarder l'augmentation de la surface de non-perfusion capillaire périphérique rétinienne (mesurable par angiographie grand champ, qui permet de mesurer un « index de non-perfusion » (ISI), qui lorsqu'il est <45% indique un risque très faible de néovascularisation (Tsui I. et al. Retina 2011;31:105-10), notamment dans les premières semaines où la maladie progresse activement,
- prévenir ou retarder l'apparition de signes indirects d'ischémie rétinienne (reperfusion de la collerette irienne, rubéose irienne, glaucome néovasculaire, abolition du réflexe pupillaire afférent, mesurables par un examen biomicroscopique du segment antérieur de l'œil),
- diminuer l'épaisseur maculaire oculaire de 100 µm, de préférence de 200 µm, de façon encore plus préférée de 300 µm après 3 mois de traitement, et/ou
- éviter ou inhiber la baisse d'acuité visuelle (perte de plus de 15 lettres / 3 lignes ETDRS).

**L'hydroxycarbamide** (HC) (Formule chimique : CH₄N₂O₂, analogue hydroxylé de l'urée - autrement appelée hydroxyurée ou HU) a été synthétisée en Allemagne en 1869 par Dressler et Stein mais ce n'est qu'en 1928 qu'elle a été administrée à des animaux chez qui on observait des anomalies sanguines à type de leucopénie, macrocytose et anémie. Il faut attendre 1963, pour que Stearns et al. montrent que l'hydroxycarbamide peut être active contre la leucémie L1210 de la souris, puis par la suite de nombreuses études cliniques ont mis en évidence son action anti tumorale chez l'homme. Certaines de ces études ont montré son efficacité dans le traitement de tumeurs solides telles que mélanome malin, cancer ovarien réfractaire, carcinome de la tête et du cou, carcinome rénal ou vésical. Mais l'apparition de nouveaux traitements plus efficaces sur ces tumeurs et le faible taux de réponse ont aujourd'hui relégué l'HC après d'autres lignes de traitement et toujours en association avec d'autres chimiothérapies.

Aujourd'hui, l'HC conserve cependant tout son intérêt dans la prise en charge thérapeutique :
- Des syndromes myéloprolifératifs tels que la polyglobulie de Vaquez (PV), la thrombocytémie essentielle (TE), la splénomégalie myéloïde ou la leucémie myéloïde chronique (le médicament HYDREA® est alors administré).

L'HC est un inhibiteur spécifique de la phase S du cycle cellulaire. Il inhibe la ribonucléotide réductase (RR) qui transforme les ribonucléotides en déoxyribonucléotides. Cette enzyme qui est produite en très petite quantité provoque par son inhibition une diminution de la production de précurseurs de l'ADN. Cet effet est dose-dépendant et corrélé également avec la durée d'exposition au médicament dans la plupart des cultures cellulaires de mammifère sans effet secondaire majeur. Cette propriété est celle principalement recherchée dans le traitement des syndromes myéloprolifératifs.
- De la prévention des crises vaso-occlusives et du syndrome thoracique aigu dans la drépanocytose symptomatique (le médicament SIKLOS® est alors administré).

L'HC est aujourd'hui le seul principe actif qui modifie le parcours de la maladie drépanocytaire. Le suivi de 17,5 ans de l'étude MSH publié par Steinberg et al (Steinberg MH et al., Am J Hematol. 2010; 85:403-8) montre que les patients qui ont reçu un traitement par HC ont un taux de mortalité inférieur de 40% à celui des patients n'ayant pas reçu HC. Par ailleurs, Voskaridou et al (Blood 2010; 115:2354-63) rapportent le suivi pendant 17 ans d'une étude prospective portant sur 330 patients. Parmi eux, 131 patients recevaient HC alors que 199 patients étaient traités de façon conventionnelle par antalgiques, hydratation, ou oxygénothérapie. La probabilité de survie globale à 10 ans était de 86% et 65% pour les receveurs d'HC et non HC respectivement (p=0.001), et ce malgré le fait que les patients traités par HC avaient des formes plus sévères de drépanocytose.

Les patients drépanocytaires ne développent pas d'OVCR, bien que d'autres complications vasculaires rétiniennes aient été observées chez ces patients (cf. Lim JI et al. Curr Opinion Ophthalmol 2012;23:533-6). De même, les patients souffrant de syndromes myéloprolifératifs ne développent pas d'OVCR.

Deux phénomènes (au moins) peuvent expliquer ces observations :
D'une part, les globules rouges (GR) des patients OVCR ne présentent pas les mêmes anomalies que les GR des patients souffrant de drépanocytose ou de syndromes myéloprolifératifs. En particulier, la plupart des molécules d'adhérence sont normalement exprimées à la surface des GR des patients OVCR (Wautier MP et al. J Thromb Haemost 2011;9:1049-55), ce qui n'est pas le cas pour les GR des patients atteints de drépanocytose ou de syndromes myéloprolifératifs (Colin Y et al. Curr Opin Hematol 2014;21:369).
D'autre part, il est décrit que les cellules endothéliales de la microcirculation de différents tissus ont des profils d'expression géniques et protéiques distincts et caractéristiques (Chi JT et al. PNAS 2003;100:10623-8).

De préférence, dans le cadre de l'invention, les patients traités (ou à traiter) ne souffrent que d'OVCR. En particulier, ils ne souffrent pas, outre l'OVCR, d'un syndrome myéloprolifératif. De plus, ils ne souffrent pas, outre l'OVCR, de drépanocytose. Enfin, d'une manière générale, ils ne souffrent d'aucune maladie générale grave et évolutive, telle qu'un cancer (solide ou leucémie).

Par « **syndrome myéloprolifératif** », on entend, au sens de la présente invention, les maladies suivantes : la polyglobulie primitive ou « polycythemia vera » (PV), la thrombocytémie essentielle (TE), la splénomégalie myéloïde, la leucémie myéloïde chronique (LMC).

En particulier, les patients traités (ou à traiter) dans le cadre de l'invention ne souffrent pas de thrombocytémie essentielle.

Par « **cancer** », on entend, au sens de l'invention, n'importe quel type de cancer solide (tel que le mélanome malin, le cancer ovarien réfractaire, le carcinome de la tête et du cou, le carcinome rénal ou vésical) ou du sang (tel que les leucémies).

En particulier, les patients traités (ou à traiter) dans le cadre de l'invention ne souffrent pas d'un glaucome.

En outre, les patients traités (ou à traiter) dans le cadre de l'invention sont de préférence des adultes (hommes ou femmes) âgés d'au minimum 45 ans. Du fait de la très faible toxicité d'HC, y compris chez les personnes âgées, il n'y a pas d'âge maximal pour être traité par le traitement de l'invention.

De préférence, les patients traités (ou à traiter) dans le cadre de l'invention ont été diagnostiqués comme souffrant d'OVCR depuis moins d'un mois. En d'autres termes, les symptômes de l'OVCR chez ledit patient sont apparus moins d'un mois, de avant ladite administration. La surface de non-perfusion rétinienne chez ces patients s'accroit alors très rapidement, d'environ 30% en 15 jours, ce qui peut entraîner une ischémie rétinienne et, à terme, leur cécité (Singer M et al. Retina 2014; 34:1736-42; Spaide RF et al. Retina 2011;31:829-37).

Le **diagnostic de l'OVCR** se fait classiquement par des examens ophtalmologiques conventionnels, réalisables chez un ophtalmologiste ou dans des hôpitaux. Il repose sur l'examen du fond d'œil. Certaines explorations complémentaires (angiographie rétinienne, tomographie par cohérence optique (OCT)) peuvent être nécessaires pour en évaluer le retentissement.

Plus précisément, les patients traités (ou à traiter) dans le cadre de l'invention auront été sélectionnés car ils présentent les symptômes suivants :
- Baisse d'acuité visuelle depuis moins d'un mois,
- Présence à l'examen du fond d'œil des signes caractéristiques d'OVCR :
   ∘ Hémorragies rétiniennes disséminées dans les 4 quadrants,
   ∘ Dilatation et tortuosité des veines,
- Si une angiographie à la fluorescéine a été réalisée : retard de remplissage veineux.

Le diagnostic de l'OACR, qui est le plus souvent due à une embolie, se fait également de manière classique par des examens ophtalmologiques conventionnels, réalisables chez un ophtalmologiste ou dans des hôpitaux. Il repose sur l'examen du fond d'œil.

Toutefois, à la différence de l'OVCR, les patients atteints de l'OACR présentent les symptômes suivants :
- Baisse de l'acuité visuelle très brutale (1 sec.) et perte totale du champ visuel ;
- Mydriase de l'œil malade ;
- L'examen du fond de l'œil sur pupilles dilatées qui permet d'établir :
   ∘ une disparition des artères grêles,
   ∘ un œdème du pôle postérieur de l'œil,
   ∘ une macula avec une couleur de type « rouge cerise ».

Le **traitement** de l'invention est tel qu'une dose comprise entre 12,5 et 25 mg/kg/jour, de préférence 20mg/kg/jour d'hydroxycarbamide est administrée auxdits patients. Ces doses sont superposables aux doses utilisées dans les syndromes myéloprolifératifs ainsi que dans la drépanocytose. A ces doses, très peu d'effets indésirables ont été rapportés chez l'adulte comme chez l'enfant.

La dose administrée peut varier dans les cas suivants:
1- en cas d'absence d'augmentation du volume globulaire moyen (augmentation <10%) après le 1^{er} mois de traitement, la dose d'HC peut être augmentée de 2.5 mg/kg/j.
2- en cas de toxicité (baisse de l'hémoglobine de plus de 20% par rapport à l'hémoglobine de base, et/ou réticulocytes < 80 000/mm³, et/ou polynucléaires neutrophiles < 2000/ mm³, et/ou plaquettes < 80 000/ mm³), le traitement peut être arrêté transitoirement et repris à une dose inférieure de 2.5 mg/kg/j par rapport à la dose précédente. En cas de toxicité récidivante à une dose inférieure à 15 mg/kg/j, le traitement doit être définitivement arrêté.

L'administration d'HC se fait de préférence par **voie orale**, ce qui la rend facile, rapide et sans danger pour le patient. Avantageusement, ce mode d'administration permet de s'affranchir de l'intervention d'un professionnel de santé à l'inverse des traitements locaux actuellement utilisés.

La présente invention vise donc l'utilisation d'HC pour préparer une composition pharmaceutique (ou un médicament) destinée à traiter par voie systémique les patients souffrant d'OVCR (tels que définis ci-dessus), plus précisément, destinée à prévenir ou retarder l'augmentation de la surface de non-perfusion capillaire rétinienne, prévenir ou retarder l'apparition de signes indirects d'ischémie rétinienne, maintenir l'épaisseur maculaire (ou la diminuer en cas d'œdème maculaire), et/ou maintenir l'acuité visuelle chez ces patients.

Plus précisément, cette composition sera destinée à prévenir, retarder voire éviter l'augmentation de la surface de non-perfusion capillaire rétinienne de manière à ce que la surface n'augmente pas de plus de 30%, de préférence de plus de 20%, de façon encore plus préférée de plus de 10%, par rapport à la surface de non-perfusion capillaire rétinienne avant traitement.

Par ailleurs, cette composition sera destinée à prévenir, retarder voire éviter la baisse de l'acuité visuelle de manière à ce que cette baisse reste inférieure à 15 lettres sur l'échelle ETDRS, de préférence inférieure à 10 lettres sur l'échelle ETDRS.

Enfin, cette composition sera destinée à diminuer l'épaisseur maculaire oculaire de 100 µm, de préférence de 200 µm, de façon encore plus préférée de 300 µm après 3 mois de traitement.

La présente invention vise également une **composition pharmaceutique** administrable par voie systémique (de préférence par voie orale), pour son utilisation pour prévenir ou traiter les patients souffrant d'OVCR (tels que définis ci-dessus), plus précisément, pour prévenir ou retarder l'augmentation de la surface de non-perfusion capillaire périphérique rétinienne, prévenir ou retarder l'apparition de signes indirects d'ischémie rétinienne, maintenir l'épaisseur maculaire, et/ou maintenir l'acuité visuelle chez ces patients.

Cette composition pharmaceutique contient, outre l'HC, au moins un excipient pharmaceutiquement acceptable.

Par « **excipient pharmaceutiquement acceptable** », on entend ici un composé ou une combinaison de composés ne provoquant pas de réactions secondaires et qui permet par exemple la facilitation de l'administration du composé actif, l'augmentation de sa durée de vie et/ou de son efficacité dans l'organisme, l'augmentation de sa solubilité en solution ou encore l'amélioration de sa conservation. Ces excipients pharmaceutiquement acceptables sont bien connus et seront adaptés par l'homme de l'art en fonction de la nature et du mode d'administration du ou des composés actifs choisis.

La forme galénique de cette composition est de préférence une forme administrable par voie orale, par exemple un comprimé, une gélule, une poudre, une granule, une solution ou une suspension orale (sirop) ou toute forme d'administration sublinguale et buccale.

Lorsque l'on prépare une composition solide sous forme de comprimés, on peut mélanger l'ingrédient actif (HC) avec un excipient pharmaceutique tel que la gélatine, l'amidon, le lactose, le stéarate de magnésium, le talc, la gomme arabique ou des analogues. On peut enrober ces comprimés de saccharose ou d'autres matières appropriées, ou encore les traiter de telle sorte qu'ils aient une activité prolongée ou retardée et qu'ils libèrent une quantité prédéterminée d'HC d'une façon continue.

On obtient des gélules par exemple en mélangeant l'ingrédient actif (HC) avec un diluant et en versant le mélange obtenu dans des gélules molles ou dures.

Une préparation sous forme de sirop ou d'élixir peut contenir l'ingrédient actif (HC) conjointement avec un édulcorant, un antiseptique, ainsi qu'un agent donnant du goût et/ou un colorant approprié.

Les poudres ou les granules dispersibles dans l'eau peuvent contenir l'ingrédient actif (HC) éventuellement en mélange avec des agents de dispersion, des agents mouillants, des agents de mise en suspension, avec des correcteurs du goût et/ou des édulcorants.

Il est par exemple possible de traiter lesdits patients à l'aide de comprimés SIKLOS 1000mg contenant, outre 1000mg d'hydroxycarbamide, du stéaryl fumarate de sodium, de la cellulose microcristalline silicifiée et du méthacrylate de butyle copolymère basique. Il est également possible de traiter lesdits patients à l'aide de comprimés HYDREA 500mg contenant, outre 500mg d'hydroxycarbamide, de l'hydrogénophosphate de sodium, de l'acide citrique, du lactose et du stéarate de magnésium.

La prise de la composition pharmaceutique de l'invention se fait de préférence à jeun, de manière encore plus préférée, le matin avant le petit déjeuner.

Cette prise a lieu de préférence tous les jours pendant au moins six mois.

La présente invention vise donc une méthode pour prévenir ou traiter des patients souffrant d'OVCR (tels que définis ci-dessus), plus précisément, pour prévenir ou retarder l'augmentation de la surface de non-perfusion capillaire périphérique rétinienne, prévenir ou retarder l'apparition de signes indirects d'ischémie rétinienne, diminuer l'épaisseur maculaire en cas d'œdème maculaire, et/ou maintenir l'acuité visuelle chez ces patients, ladite méthode comprenant l'étape d'administrer au dit patient, par voie systémique, une quantité efficace d'une composition pharmaceutique telle que définie ci-dessus, contenant de l'hydroxycarbamide.

### Description des figures

La Figure 1 représente l'acuité visuelle (AV) et le volume maculaire moyen (OCT) du patient la veille du traitement (J-1), ainsi que 15 jours après le début du traitement (S2), 27 jours après le début du traitement (S4) et 107 jours après le début du traitement (S13).
La Figure 2 représente (**A**) le fond d'œil (FO), l'angiographie rétinienne à la fluorescéine (FA) et (**B**) l'angio-OCT présentant la surface de la zone avasculaire centrale (ZAC) la veille du traitement (J-1), ainsi que 27 jours après le début du traitement (S4) et 107 jours après le début du traitement (S13).

### Exemples

### 1. Patients étudiés dans l'étude

Les patients enrôlés dans l'étude sont des personnes âgées d'au moins 45 ans, des deux sexes, présentant une OVCR dont les symptômes sont apparus moins d'un mois avant leur prise en charge, et comprennent une baisse d'acuité visuelle avec acuité visuelle corrigée de l'œil atteint inférieure à 6/10 (73 lettres ETDRS).

Les patients ne remplissant pas ces critères ne sont pas intégrés à l'étude. En outre, sont exclus les patient(e)s :
- Monophtalmes (dont l'acuité visuelle controlatérale inférieure à 4/10),
- Ayant un antécédent de traumatisme ou de chirurgie oculo-orbitaire dans les 6 mois précédant les premières manifestations cliniques ophtalmologiques,
- Ayant un antécédent de radiothérapie céphalique,
- Souffrant d'une tumeur orbitaire ou du nerf optique homolatérale à l'OVR,
- Souffrant d'une pathologie rétinienne vasculaire controlatérale, en particulier rétinopathie diabétique modérée (le stade minime n'étant pas une contre-indication),
- Souffrant d'une néovascularisation oculaire (dont rubéose irienne),
- Souffrant d'une contre-indication à la dilatation pupillaire (dont allergie au tropicamide),
- Allergique à la fluorescéine, ou à l'HC,
- Souffrant d'une pathologie locale ou générale inflammatoire évolutive,
- Souffrant de drépanocytose,
- Souffrant d'un syndrome myéloprolifératif connu,
- - Souffrant de signes de myélosuppression : hémoglobine < 13 g/dL (homme) ou < 12 g/dL (femme), réticulocytes < 20000/mm³, polynucléaires neutrophiles < 1500/mm³, plaquettes < 150000/mm³
- Etant traité par hydroxycarbamide avant l'inclusion au protocole,
- Etant traité par anticoagulant avant l'inclusion au protocole,
- Souffrant d'insuffisance rénale avec clairance de la créatinine ≤ 60 ml/min,
- Souffrant d'insuffisance hépatique sévère (classification de Child Pugh C),
- Les femmes enceintes ou allaitantes ou sans contraception,
- Les hommes ayant un désir de paternité.

### 2. Protocole de suivi et d'administration du médicament

Entre J0-10 et J0 ou entre J0-20 et J0 : un médecin vérifie l'ensemble des critères d'éligibilité et de non-éligibilité au protocole, recueille l'observation clinique complète du patient, et lui donne une information orale détaillée et interactive sur l'étude, ainsi que la notice d'information et la fiche de consentement.

Des examens sanguins (une mesure de la NFS et de la créatininémie) sont par ailleurs effectués. Ces examens font partie du bilan usuel d'OVCR, et permettent de vérifier l'absence de critère biologique de non inclusion dans l'étude.

J0 : Il s'agit du jour d'inclusion du patient dans l'étude. Ce même jour, après que le patient a signé un consentement écrit, les tests suivants sont réalisés:
- Prélèvements sanguins (NFS-réticulocytes, VS, CRP, TP, TCA, fibrine, électrophorèse des protides, créatininémie, bilan lipidique, glycémie à jeun, HbA1c). Pour ces différents tests, le patient est à jeun.
- Examens ophtalmologiques spécialisés (angiographie rétinienne, OCT).

Le patient reçoit par ailleurs les comprimés d'hydroxycarbamide lui permettant d'effectuer la première partie du traitement. La première prise du traitement doit être effectuée par le patient dès le lendemain matin (J1), à jeun. Le traitement doit ensuite être pris tous les jours, de préférence à jeun.

La forme galénique utilisée dans la présente étude est la forme orale SIKLOS commercialisée par le Laboratoire Addmedica en France. Ce médicament a reçu une AMM européenne le 28/06/2007 pour la spécialité SIKLOS 1000 mg comprimé pelliculé dans la prévention des crises vaso-occlusives douloureuses récurrentes y compris celle du syndrome thoracique aigu chez l'adulte, l'adolescent et l'enfant âgé de plus de 2 ans souffrant de drépanocytose symptomatique. C'est un comprimé oblong, blanc, pelliculé, trisécable permettant de fournir 4 parts égales de 250 mg. Ces comprimés sont présentés en flacon de polyéthylène haute densité (HDPE) contenant 30 comprimés avec un bouchon sécurité-enfant en polypropylène (PP) avec dessicant. Il doit être stocké dans un lieu dont la température ne dépasse pas 30°C pour une durée maximale de 24 mois. Chaque comprimé de SIKLOS 1000 mg contient 1000 mg d'hydroxycarbamide. La composition qualitative de Siklos 1000 mg est donc hydroxycarbamide, stéaryl fumarate de sodium, cellulose microcristalline silicifiée et méthacrylate de butyle copolymère basique.

Selon le protocole, le patient doit avaler l'équivalent de 20mg/kg du comprimé SIKLOS tous les jours pendant un mois. Le tableau ci-dessous indique la dose et le nombre de comprimés de SIKLOS à prendre en fonction du poids corporel du patient.

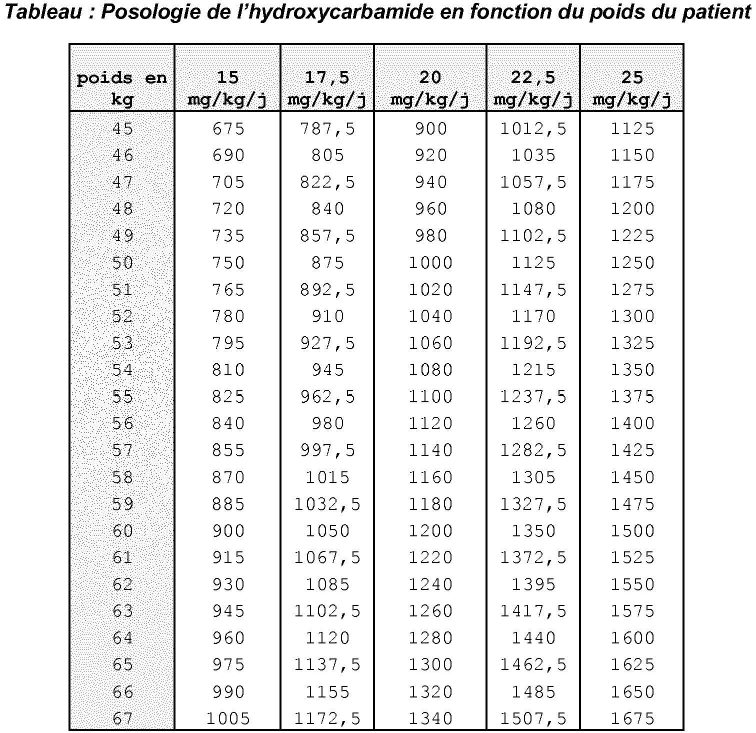

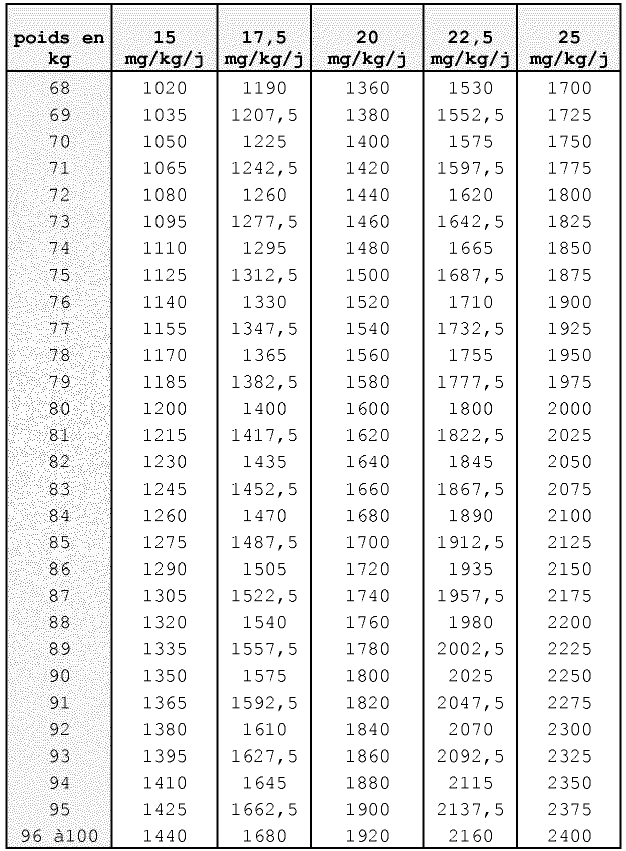

Il est conseillé aux patients de prendre le médicament de préférence le matin avant le petit déjeuner (à jeun), si besoin dans une petite quantité d'eau ou de nourriture. Pour les patients incapables d'avaler les comprimés, il est possible de les dissoudre immédiatement avant leur prise, dans une petite cuillère avec un peu d'eau.

A J15 (S2), J30 (S4), J45, J60 ainsi qu'à M3 (S13), M4, M5 et M6 (S26) : un bilan hématologique (NFS, réticulocytes) est réalisé, pour surveiller la tolérance hématologique du traitement.

Les visites de suivi ont lieu à J15 (S2), M1 (S4), M3 (S13) et M6 (S26) (J15 : 14±2, M1 : 28±2, M3 : 91±20 jours, M6 : 182±2 jours après le début du traitement). Elles comportent un examen ophtalmologique, une consultation en médecine interne, des prélèvements sanguins au laboratoire, et un passage à la pharmacie pour comptabilisation du traitement pris et délivrance du lot suivant.

Les 3 visites de médecine réalisées à M1 (S4), M3 (S13) et M6 (S26) ont comme objectif principal d'évaluer l'observance et la tolérance du traitement tout au long de l'étude.

L'efficacité du traitement en ce qui concerne la prévention de la non-perfusion rétinienne est évaluée à J15 (S4), M1 (S13) et M6 (S26). L'effet du traitement sur l'acuité visuelle et sur l'épaisseur maculaire est évalué à J15 (S2), M1 (S4), M3 (S13) et M6 (S26). A M6 (S26), l'incidence de rubéose irienne et de glaucome néovasculaire est évaluée.

L'efficacité du traitement sur les paramètres biologiques de la recherche, en particulier sur les paramètres hémorhéologiques *in vitro* est évaluée à J15 (S2), M1 (S4), M3 (S13) et M6 (S26), et la pousse spontanée des colonies érythroides est testée à M3 (S13).

Un changement de dose est fait dans les cas suivants:
1- en cas d'absence d'augmentation du volume globulaire moyen (augmentation <10%) après le 1^{er} mois de traitement, la dose d'HC a été augmentée de 2.5 mg/kg/j.
2- en cas de toxicité (baisse de l'hémoglobine de plus de 20% par rapport à l'hémoglobine de base, et/ou réticulocytes < 80 000/mm³, et/ou polynucléaires neutrophiles < 2000/ mm³, et/ou plaquettes < 80 000/ mm³), le traitement est arrêté transitoirement et repris à une dose inférieure de 2.5 mg/kg/j par rapport à la dose précédente. En cas de toxicité récidivante à une dose inférieure à 15 mg/kg/j, le traitement est définitivement arrêté.

### 3. Tests effectués

Le bilan sanguin réalisé à J0 comprend la mesure de : NFS-réticulocytes, VS, CRP, TP, TCA, fibrine, électrophorèse des protides, créatininémie, bilan lipidique, glycémie à jeun, HbA1c.

Ce bilan vise à éliminer la présence d'une éventuelle hémopathie, en particulier un syndrome myéloprolifératif, ainsi qu'une éventuelle insuffisance hépatique ou rénale sévère, constituant autant de critères de non inclusion dans l'étude. Il vise aussi à préciser les facteurs de risque vasculaire du patient, diabète et dyslipidémie notamment.

Le même jour (à J0), les patients subissent un bilan ophtalmologique complet, comprenant une rétinophotographie rétinienne, une acuité visuelle ETDRS, une angiographie et un examen OCT.

Les examens ophtalmologiques réalisés dans le cadre de cette étude font partie de l'exploration en routine des OVCR.

La prise de clichés du fond d'œil (rétinophotographie) permet d'obtenir un document d'archive.

L'angiographie à la fluorescéine est effectuée avec un ophtalmoscope laser à balayage (SLO) de type Spectralis (Heidelberg Engineering, Heidelberg, Allemagne), pourvu d'un objectif « ultra-grand champ » (UWF 102). Lors de l'imagerie proprement dite, le patient, dont la pupille a été dilatée au préalable, est invité à poser la tête sur une mentonnière. Une fois la mise au point effectuée, il est procédé à l'injection intraveineuse de fluorescéine. Le fond d'œil est surveillé en continu pour détecter l'apparition de fluorescéine dans les artères rétiniennes. Dès l'apparition de la fluorescéine dans les artères rétiniennes, une acquisition vidéo à haute résolution pendant toute la durée du remplissage vasculaire (soit en moyenne 10 secondes) est déclenchée. Dès le remplissage artério-veineux complété, un cliché au plus fort grossissement, centré sur la zone avasculaire rétinienne (ZAC), est réalisé (aux deux yeux), puis le reste de l'angiographie est poursuivi en grand champ. La surface de non-perfusion est mesurée manuellement à l'aide des outils de mesure de l'appareil Spectralis, en faisant le rapport de la surface non perfusée sur la surface totale de rétine.

L'OCT comprend deux coupes (horizontale et verticale) centrées sur la fovea, ainsi qu'une cartographie maculaire. Une angio-OCT est également réalisée à l'aide de l'appareil Optovue® pour évaluer la maille capillaire fovéolaire sans injection. La surface de la zone avasculaire centrale (ZAC) est ensuite mesurée à l'aide des outils logiciels fournis par le constructeur.

La mesure standardisée de l'acuité visuelle par l'échelle ETDRS, internationalement reconnue et validée, est effectuée par une orthoptiste (durée 30 minutes environ). Cette mesure est réalisée à chaque visite des patients de l'étude.

L'angiographie à la fluorescéine étant parfois peu contributive ou difficile à interpréter, l'évolution de la perfusion rétinienne est évaluée non seulement en angiographie (modification de la surface de rétine non perfusée et de la zone avasculaire centrale), mais aussi en recherchant la survenue de signes cliniques secondaires à l'ischémie rétinienne : abolition du réflexe pupillaire afférent, dilatation de vaisseaux iriens, reperfusion de la collerette irienne, apparition d'une néovascularisation irienne, dans l'angle irido-cornéen ou pré-rétinienne, voire survenue d'un glaucome néovasculaire.

Des tests sont également réalisés pour comprendre le mécanisme d'action de l'HC sur les capillaires rétiniens. En particulier, les progéniteurs hématopoïétiques circulants sont prélevés (2 tubes 5ml sur EDTA) à J0 puis à M3 (S13) pour les patients ayant des colonies érythroides endogènes à l'inclusion. Les propriétés rhéologiques des globules rouges sont analysées sur un ektacytomètre LoRRca MaxSis Osmoscan, (Mechatronics) et un viscosimètre Brookfield. Plusieurs caractéristiques sont étudiées : la viscosité sanguine, l'agrégation globale des globules rouges dans le plasma des patients, mais aussi les caractéristiques de stabilité et de déformabilité membranaire.

### 4. Résultats

Après analyse des tests ophtalmiques décrits ci-dessus, le taux de réussite du traitement est proportionnel au pourcentage de patients chez qui aucun des évènements suivants n'a été observé trois mois après le début du traitement :
- Augmentation de la surface de non-perfusion périphérique mesurée en angiographie grand champ de plus de 30%,
- Apparition de signes indirects d'ischémie rétinienne : reperfusion de la collerette irienne, rubéose irienne, glaucome néovasculaire, diminution du réflexe pupillaire afférent,

En outre, les tests sanguins sont importants pour contrôler la tolérance hématologique du traitement.

Les résultats de l'étude clinique ci-dessous montrent qu'il est possible de réduire l'augmentation de la surface de non-perfusion rétinienne en administrant par voie orale de l'HC dans les conditions de l'étude. En outre, le traitement oral par HC aux doses utilisées se révèle, conformément aux précédentes études cliniques réalisées avec ce traitement, très peu toxique.

### Etude clinique

Le cas d'un homme âgé de 60 ans, d'origine caucasienne, aux antécédents déjà anciens de maladie des griffes du chat et d'érysipèle de jambe gauche, sans facteur de risque vasculaire et sans traitement chronique, ayant consulté aux urgences ophtalmologiques pour des métamorphopsies et un flou visuel de l'œil gauche persistant depuis 24h, et recevant le traitement conformément au protocole présenté ci-dessus, a été suivi comme décrit ci-dessous.

L'examen ophtalmologique initial du patient révélait une acuité visuelle gauche de 10/10f P2 et une pression intraoculaire de 15mmHg, avec au fond d'œil une hémi-occlusion inférieure de la veine centrale de la rétine, avec dilatation et tortuosité veineuse, hémorragies et nodules cotonneux.

Treize jours après sa première visite, l'examen ophtalmologique révélait une acuité visuelle de l'œil gauche de 5/10f P2 et une pression intraoculaire de 16mmHg. La tomographie en cohérence optique (OCT) montrait un œdème maculaire et une hémorragie juxta-fovéolaire. Dix-neuf jours après sa première visite (J-1 de traitement), l'acuité visuelle mesurée sur l'échelle ETDRS était de 71 lettres pour l'œil gauche et de 89 lettres pour l'œil droit. L'angiographie rétinienne à la fluorescéine a confirmé le diagnostic d'OVCR œdémateuse en retrouvant un ralentissement circulatoire de l'hémi-veine inférieure et l'absence de zone de non perfusion. Une OCT-angiographie a permis d'évaluer la surface de la zone avasculaire centrale (ZAC) égale à 0,12 mm². L'épaisseur centrale moyenne de la surface rétinienne était égale à 316µm et le volume maculaire moyen à 6,12 mm³ (Figures 1 et 2, J-1).

Le traitement par hydroxycarbamide est débuté le lendemain sous la forme de 1750 mg par jour (20 mg/kg/jour) de SIKLOS per os.

Les visites de suivi ont eu lieu 13 jours (S2/J15), 27 jours (S4/M1) et 108 jours (S13/M3) après le début du traitement. L'hydroxycarbamide a été stoppée pendant 3 jours (juste après la visite de S4/M1) en raison d'une thrombopénie à 60000/mm3, puis reprise à la dose de 17,5 mg/kg/J. Elle a de nouveau été stoppée 1 semaine plus tard pendant 16 jours en raison d'un taux d'hémoglobine abaissé de plus de 20% par rapport au taux d'hémoglobine avant traitement, puis reprise à la dose de 15 mg/Kg/J jusqu'à la visite de S13/M3. La tolérance clinique du traitement a été parfaite et les modifications de la numération sanguine étaient asymptomatiques.

A la visite de S2, l'acuité visuelle (échelle ETDRS) de l'œil gauche était mesurée à 73 lettres pour l'œil gauche (Figure 1). A l'examen du fond d'œil gauche était notée une diminution des hémorragies. L'OCT a montré une épaisseur centrale moyenne de la rétine de 300µm et un volume maculaire moyen de 5,6 mm³.

A la visite de S4 après le début du traitement, l'acuité visuelle (échelle ETDRS) de l'œil gauche était mesurée à 77 lettres pour l'œil gauche (Figure 1). A l'examen du fond d'œil gauche était notée une diminution des hémorragies, ainsi que du calibre et de la tortuosité veineuse inférieure (Figure 2A). L'OCT a montré une épaisseur centrale moyenne de la rétine de 293µm et un volume maculaire moyen de 5,48mm³ (Figure 1). L'angiographie rétinienne à la fluorescéine retrouvait une amélioration des temps circulatoires dans l'hémi-veine inférieure avec l'apparition d'une collatérale papillaire et confirmait l'absence de territoire de non-perfusion (Figure 2A). En OCT-angiographie, la surface de la zone avasculaire centrale (ZAC) était évaluée à 0,10 mm² (Figure 2B).

A la visite de S13 après le début du traitement, l'acuité visuelle gauche était mesurée à 82 lettres (ETDRS ; Figure 1). A l'examen du fond d'œil gauche étaient notés une disparition des hémorragies, un calibre veineux inférieur quasi-normal et la présence d'une circulation collatérale papillaire (Figure 2A). En OCT, le profil maculaire était sensiblement normal, avec un volume maculaire moyen de 5,23 mm³ (Figure 1). L'épaisseur centrale moyenne de la rétine était de 281 µm. L'angiographie rétinienne à la fluorescéine retrouvait une normalisation des temps circulatoires dans l'hémi-veine inférieure et confirmait l'absence de territoire de non-perfusion (Figure 2A). L'OCT-angiographie a permis d'évaluer la surface de la zone avasculaire centrale (ZAC) égale à 0,09 mm² (Figure 2B).

En conclusion, il a été observé chez ce patient âgé de 60 ans atteint d'une hémi-occlusion veineuse, sous traitement par hydroxycarbamide, une amélioration de la circulation dans l'hémi-veine inférieure ainsi que le développement d'une circulation de suppléance, la normalisation du calibre veineux et du FO avec la disparition des hémorragies et la diminution de l'œdème maculaire permettant une amélioration de l'acuité visuelle (+11 lettres ETDRS). Il a également été observé une diminution de l'épaisseur centrale moyenne de la rétine modérée (diminution de 35µm), l'œdème n'étant pas central chez ce patient. De manière importante, la rétine périphérique est toujours restée bien perfusée au cours du traitement et la surface de la ZAC n'a pas été significativement modifiée.

## Revendications

1. Hydroxycarbamide (HC) pour son utilisation pour prévenir l'augmentation de la surface de non-perfusion capillaire chez un patient souffrant d'occlusion de veine centrale de la rétine (OVCR), **caractérisée en ce que** son administration s'effectue par voie systémique.

2. Hydroxycarbamide pour son utilisation selon la revendication 1, **caractérisée en ce que** ledit patient ne souffre pas d'un syndrome myéloprolifératif.

3. Hydroxycarbamide pour son utilisation selon la revendication 1 ou 2, **caractérisée en ce que** ledit patient ne souffre pas de drépanocytose.

4. Hydroxycarbamide pour son utilisation selon l'une des revendications 1 à 3, **caractérisée en ce que** ledit patient est une personne âgée d'au moins 45 ans.

5. Hydroxycarbamide pour son utilisation selon l'une des revendications 1 à 4, **caractérisée en ce que** les symptômes de l'OVCR chez ledit patient sont apparus moins d'un mois avant ladite administration.

6. Hydroxycarbamide pour son utilisation selon l'une des revendications 1 à 5, **caractérisée en ce qu'**une dose de 20mg/kg/ jour est administrée audit patient.

7. Hydroxycarbamide pour son utilisation selon l'une des revendications 1 à 6, **caractérisée en ce que** son administration s'effectue par voie orale.

8. Hydroxycarbamide pour son utilisation selon l'une des revendications 1 à 7, **caractérisé en ce que** l'augmentation de la surface de non-perfusion capillaire est maintenue inférieure à 30 %, de préférence 20 %, de façon encore plus préférée 10 %.

9. Hydroxycarbamide pour son utilisation selon l'une des revendications 1 à 8, **caractérisé en ce qu'**une baisse de l'acuité visuelle est évitée.

10. Hydroxycarbamide pour son utilisation selon l'une des revendications 1 à 8, **caractérisé en ce que** l'épaisseur maculaire est diminuée de 100µm après 3 mois de traitement.

## Patentansprüche

1. Hydroxycarbamid (HC) zur Verwendung zur Vorbeugung der Vergrößerung der Fläche einer retinalen Ischämie bei einem Patienten, der an einem retinalen Zentralvenenverschluss leidet, **dadurch gekennzeichnet, dass** seine Verabreichung auf systemischem Weg erfolgt.

2. Hydroxycarbamid zur Verwendung nach Anspruch 1, **dadurch gekennzeichnet, dass** der Patient nicht an einem myeloproliferativen Syndrom leidet.

3. Hydroxycarbamid zur Verwendung nach Anspruch 1 oder 2, **dadurch gekennzeichnet, dass** der Patient nicht an Sichelzellenanämie leidet.

4. Hydroxycarbamid zur Verwendung nach einem der Ansprüche 1 bis 3, **dadurch gekennzeichnet, dass** der Patient eine Person in einem Alter von mindestens 45 Jahren ist.

5. Hydroxycarbamid zur Verwendung nach einem der Ansprüche 1 bis 4, **dadurch gekennzeichnet, dass** die Symptome des retinalen Zentralvenenverschlusses beim Patienten weniger als einen Monat vor der Verabreichung aufgetreten sind.

6. Hydroxycarbamid zur Verwendung nach einem der Ansprüche 1 bis 5, **dadurch gekennzeichnet, dass** dem Patienten eine Dosis von 20 mg/kg/Tag verabreicht wird.

7. Hydroxycarbamid zur Verwendung nach einem der Ansprüche 1 bis 6, **dadurch gekennzeichnet, dass** seine Verabreichung auf oralem Weg erfolgt.

8. Hydroxycarbamid zur Verwendung nach einem der Ansprüche 1 bis 7, **dadurch gekennzeichnet, dass** die Vergrößerung der Fläche der retinalen Ischämie kleiner als 30%, vorzugsweise als 20%, noch mehr zu bevorzugen als 10% gehalten wird.

9. Hydroxycarbamid zur Verwendung nach einem der Ansprüche 1 bis 8, **dadurch gekennzeichnet, dass** eine Verminderung der Sehschärfe verhindert wird.

10. Hydroxycarbamid zur Verwendung nach einem der Ansprüche 1 bis 8, **dadurch gekennzeichnet, dass** die Makuladicke nach 3 Monaten Behandlung um 100 µm verringert ist.

## Claims

1. Hydroxycarbamide (HC) for use in preventing the increase in capillary non-perfusion area in a patient with central retinal vein occlusion (CRVO), **characterized in that** it is administered systemically.

2. Hydroxycarbamide for use according to claim 1, **characterized in that** said patient does not suffer from myeloproliferative syndrome.

3. Hydroxycarbamide for use according to claim 1 or 2, **characterized in that** said patient does not suffer from sickle cell disease.

4. Hydroxycarbamide for use according to one of claims 1 to 3, **characterized in that** said patient is a person aged at least 45 years.

5. Hydroxycarbamide for use according to one of claims 1 to 4, **characterized in that** the symptoms of CRVO in said patient appeared less than one month before said administration.

6. Hydroxycarbamide for use according to one of claims 1 to 5, **characterized in that** a dose of 20 mg/kg/day is administered to said patient.

7. Hydroxycarbamide for use according to one of claims 1 to 6, **characterized in that** it is administered orally.

8. Hydroxycarbamide for use according to one of claims 1 to 7, **characterized in that** the increase in capillary non-perfusion area is maintained below 30%, preferably 20%, even more preferably 10%.

9. Hydroxycarbamide for use according to one of claims 1 to 8, **characterized in that** a decrease in visual acuity is avoided.

10. Hydroxycarbamide for use according to one of claims 1 to 8, **characterized in that** macular thickness is reduced by 100 µm after 3 months of treatment.
